# EUROPÄISCHE PATENTANMELDUNG

(11) **EP 4 019 053 A1**
(43) Veröffentlichungstag der Anmeldung: **29.06.2022**
(21) Anmeldenummer: 21210502.7
(22) Anmeldetag: 25.11.2021
(51) Int. Cl.: A61L 2/10, A61L 2/24

(54) **VORRICHTUNG ZUR DESINFEKTION VON PAKETEN ODER STÜCKGUT**

(30) Priorität: 27.11.2020 DE 102020131487
(71) Anmelder: Lippert GmbH & Co. KG, 92690 Pressath (DE)
(72) Erfinder: Schug, Hubert, 92676 Eschenbach (DE)
(74) Vertreter: Louis Pöhlau Lohrentz

(57) **Zusammenfassung**

Vorgeschlagen wird eine Vorrichtung (1) zum Desinfizieren und/oder Sterilisieren von Paketen oder Stückgut mittels UV-Licht, umfassend einen UV-Tunnel (2) und ein durch den UV-Tunnel (2) in einer Transportrichtung hindurch geführtes Transportband (3) zum Transport von Paketen oder Stückgut, wobei innerhalb des UV-Tunnels (2) mehrere UV-Lichtquellen (7) angeordnet sind, und wobei der UV-Tunnel (2) einen Rahmen (4) mit einem in Transportrichtung gesehenen unteren Rahmenabschnitt (40), einem oberen Rahmenabschnitt (41), einem linken Rahmenabschnitt (42) und einem rechten Rahmenabschnitt (43) aufweist, und die Rahmenabschnitte das Transportband (3) an vier Seiten umschließen. Wesentlich dabei ist, dass in oder an dem Rahmen (4) eine durch einen öffenbaren oder abnehmbaren Deckel (5) verschlossene Wartungsöffnung (6) ausgebildet ist, wobei der Deckel (5) derart ausgebildet und angeordnet ist, dass er in geschlossenem Zustand die Wartungsöffnung (6) vollständig verschließt und in geöffnetem oder abgenommenen Zustand einen Zugriff auf eine oder mehrere oder alle UV-Lichtquellen (7) und/oder einen Austausch einer oder mehrerer oder aller UV-Lichtquellen (7) ermöglicht, und dass die mehreren UV-Lichtquellen (7) die Pakete von vier Seiten mit UV-Licht bestrahlen.

## Beschreibung

Die Erfindung betrifft eine Vorrichtung zum Desinfizieren und/oder Sterilisieren von Paketen oder Stückgut mittels UV-Licht (Ultraviolettem Licht) nach den Merkmalen des Oberbegriffs des Anspruchs 1.

Systeme zum Desinfizieren und/oder Sterilisieren von Briefen oder Paketen sind aus der Praxis bereits bekannt, wobei in diesen Systemen eine Bestrahlung mit UV-Licht immer mit einer zweiten Methode, beispielsweise einer Bestrahlung mit Röntgenstrahlen oder Mikrowellen oder mit der Benutzung von Ozon-Gas kombiniert wird, wie beispielsweise in der US 7,553,446 B oder der US 2003/01323398 A beschrieben ist. Weiter können bekannte Vorrichtungen nur sehr schwer gewartet werden, da die UV-Leuchtmittel zum Schutz der Umgebung an schwer zugänglichen Stellen angeordnet sind.

Der vorliegenden Erfindung liegt die Aufgabe zugrunde, eine verbesserte, einfachere und kostengünstigere Vorrichtung zum Desinfizieren und/oder Sterilisieren von Paketen oder Stückgut mittels UV-Licht bereitzustellen. Insbesondere soll die Vorrichtung einen zuverlässigen Betrieb und eine einfache Wartung ermöglichen.

Die Aufgabe wird erfindungsgemäß durch eine Vorrichtung zum Desinfizieren und/oder Sterilisieren von Paketen oder Stückgut mittels UV-Licht nach den Merkmalen des Anspruchs 1 gelöst.

Erfindungsgemäß wird eine Vorrichtung zum Desinfizieren und/oder Sterilisieren von Paketen oder Stückgut mittels UV-Licht vorgeschlagen, umfassend einen UV-Tunnel und ein durch den UV-Tunnel in einer Transportrichtung hindurch geführtes Transportband zum Transport von Paketen oder Stückgut, wobei innerhalb des UV-Tunnels mehrere UV-Lichtquellen angeordnet sind, und wobei der UV-Tunnel einen Rahmen mit einem in Transportrichtung gesehenen unteren Rahmenabschnitt, einem oberen Rahmenabschnitt, einem linken Rahmenabschnitt und einem rechten Rahmenabschnitt aufweist, und die Rahmenabschnitte das Transportband an vier Seiten umschließen. Wesentlich dabei ist, dass in oder an dem Rahmen eine durch einen öffenbaren oder abnehmbaren Deckel verschlossene Wartungsöffnung ausgebildet ist, wobei der Deckel derart ausgebildet und angeordnet ist, dass er in geschlossenem Zustand die Wartungsöffnung vollständig verschließt und in geöffnetem oder abgenommenen Zustand einen Zugriff auf eine oder mehrere oder alle UV-Lichtquellen und/oder einen Austausch einer oder mehrerer oder aller UV-Lichtquellen ermöglicht, und dass die mehreren UV-Lichtquellen das Paket oder das Stückgut von vier Seiten mit UV-Licht bestrahlen.

Der Vorteil der Erfindung liegt im einfachen Aufbau der Vorrichtung. Zur Reparatur oder zur Anpassung der Beleuchtungsstärke können die UV-Lichtquellen durch einfaches Öffnen des Deckels ausgetauscht, gesäubert oder ersetzt werden. Ein mechanisch aufwändiger und zeitaufwändiger Umbau zur Reparatur oder zur Anpassung entfällt vollständig. Auch könne die Ausfallzeiten zwecks Wartung oder Reparatur der Anlage verringert werden.

Der Vorteil einer Bestrahlung von vier Seiten zusammen mit dem Transport durch den UV-Tunnel hindurch ist, dass das Paket oder das Stückgut von allen Seiten bestrahlt wird, d.h. ein Drehen des Pakets oder des Stückguts zum vollständigen und sicheren Desinfizieren und/oder Sterilisieren ist nicht notwendig. Die Transportrichtung in der Vorrichtung ist durch das Transportband vorgegeben.

Desinfektion bedeutet eine Reduzierung der Anzahl krankmachender Keime, so dass von dem Paket oder dem Stückgut keine Infektionsgefahr mehr ausgeht. Sterilisation bedeutet, dass das Paket oder das Stückgut frei von vermehrungsfähigen Keimen ist, beispielsweise von Bakterien, Pilzen, Sporen, Viren, und/oder Prionen.

UV-Licht bedeutet vorzugsweise Licht, d.h. elektromagnetische Strahlung, im Bereich von 100 nm bis 380 nm, vorzugsweise Licht im UV-C Bereich von 100 nm bis 280 nm und/oder im UV-B Bereich von 280 nm bis 315 nm und/oder im UV-A Bereich von 315 nm bis 380 nm.

Es kann vorgesehen sein, dass die UV-Lichtquelle in einem Frequenzbereich von 256, 4 nm ± 10 nm strahlt. Durch Bestrahlung in diesem Wellenlängenbereich kann ein besonders gutes Desinfizieren und/oder Sterilisieren sichergestellt werden.

UV-Tunnel bedeutet vorzugsweise, dass das Transportband über den Längenabschnitt des UV-Tunnels in Transportrichtung des Transportbands gesehen an vier Seiten (oben, unten, links und rechts) vom UV-Tunnel umschlossen ist. Der UV-Tunnel weist vorzugsweise durch die vier Seitenwände, d.h. unterer Rahmenabschnitt, oberer Rahmenabschnitt, linker Rahmenabschnitt und rechter Rahmenabschnitt, eine rechteckige Form auf. Andere Querschnitte, z.B. rund oder elliptisch, sind auch denkbar. An der Innenseite der Rahmenabschnitte des UV-Tunnels wird in Transportrichtung gesehen eine innere Querschnittsfläche mit Höhe h und Breite b ausgebildet. Eine lichte Höhe *Ih* des Tunnels wird durch die Oberfläche des Transportbandes und den oberen Rahmenabschnitt definiert. Eine untere lichte Höhe *ulh* wird durch die Oberfläche des Transportbands und dem unteren Rahmenabschnitt definiert.

Insbesondere ist vorgesehen, dass bei der erfindungsgemäßen Vorrichtung UV-Licht die einzige Methode zur Desinfektion oder Sterilisierung von Paketen oder Stückgut ist. Aufgrund der effizienten Bestrahlung der Pakete oder des Stückguts innerhalb des UV-Tunnels mit UV-Licht, ist eine weitere Methode oder ein weiteres Reagenz oder eine Generierung weiterer Strahlen oder Oxidationsmittel zur Desinfektion oder Sterilisierung nicht zwingend notwendig.

Rahmen bedeutet vorzugsweise, eine tragende Struktur bestehend aus mehreren miteinander verbundenen Profilen. Insbesondere können der untere Rahmenabschnitt, der obere Rahmenabschnitt, der linke Rahmenabschnitt und der rechte Rahmenabschnitt als tragende Elemente ausgebildet sein. Zwischenräume der Rahmenabschnitte können Wände aufweisen, insbesondere beplankt sein, um einen geschlossenen, insbesondere lichtdichten Tunnel auszubilden. Zusammen können die Rahmenabschnitte einen rechteckigen Tunnel ausbilden, der sich in Transportrichtung erstreckt.

Es kann vorgesehen sein, dass die Rahmenabschnitte des Rahmens aus festem Material, beispielsweise Aluminium oder Stahl, ausgebildet sind. Der Rahmen und/oder die einzelnen Rahmenabschnitte können aus einem Gestell und Zwischenwänden ausgebildet sein. Vorzugsweise kann die eine Wartungsöffnung oder die mehreren Wartungsöffnungen in einer oder in mehreren Zwischenwänden in oder an dem Rahmen oder an den Zwischenwänden oder Beplankungen ausgebildet sein. Vorzugsweise weist der Rahmen oder das Gestell Hohlprofile auf, um das Gewicht bei gleichbleibender Stabilität zu reduzieren.

Es kann vorgesehen sein, dass ein Paket oder dass Paketsendungen als ein Brief oder als mehrere Briefe und/oder als einzelnes Paket oder als mehrere Pakete ausgebildet sind.

Unter Stückgut ist vorzugweise jedes Transportgut zu verstehen, welches einzeln am Stück transportiert werden kann, beispielsweise Maschinen oder Anlagenteile, Kisten, Gebinde, oder Fässer, oder einzelne Bauteile.

Vorzugsweise wird das Paket und/oder das Stückgut auf dem Transportband durch den UV-Tunnel hindurch transportiert, insbesondere kontinuierlich, d.h. mit einer definierten oder einer konstanten Geschwindigkeit, insbesondere ohne abzubremsen oder anzuhalten. Insbesondere stimmt die Geschwindigkeit einer Paketsendung und/oder eines Stückguts in dem UV-Tunnel mit der Transportgeschwindigkeit derselben außerhalb des UV-Tunnels überein. Dies bedeutet, dass eine Sterilisierung in Echtzeit ermöglicht wird, ohne dass der Durchsatz einer an die Vorrichtung zum Desinfizieren und/oder Sterilisieren von Paketen oder Stückgut mittels UV-Licht angeschlossenen Sortieranlage, Verteilanlage oder Kommissionieranlage verringert wird.

Vorzugsweise sind mehrere Paketsendungen hintereinander auf dem Transportband angeordnet. Höchst vorzugsweise sind die Paketsendungen mit einem Abstand von mindestens 0,2 m, vorzugsweise mindestens 0,3 m, höchst vorzugsweise mindestens 0,5 m hintereinander angeordnet. Durch den Abstand wird eine Bestrahlung aller Seiten mehrerer hintereinander angeordneter Paketsendungen sichergestellt.

Vorzugsweise ist vor oder in dem UV-Tunnel eine Vorrichtung zum Vereinzeln angeordnet, d.h. um die Pakete und/oder das Stückgut räumlich voneinander zu trennen, vorzugsweise räumlich derart voneinander zu trennen, um die Pakete oder das Stückgut in Transportrichtung mit einem vorgegebenen oder vorgebbaren Mindestabstand in Transportrichtung anzuordnen.

Es kann vorgesehen sein, dass die UV-Lichtquellen innerhalb des UV-Tunnels an der Innenseite eines Deckels angeordnet sind, vorzugsweise mit der Innenseite des Deckels mechanisch verbunden sind. Alternativ oder ergänzend können die UV-Lichtquellen im UV-Tunnel an der Innenseite des Rahmens angeordnet sein, vorzugsweise am unteren Rahmenabschnitt, am oberen Rahmenabschnitt, am linken Rahmenabschnitt und/oder am rechten Rahmenabschnitt angeordnet sein.

An der Innenseite des Deckels und/oder des Rahmens angeordnet bedeutet hier insbesondere, dass die UV-Lichtquellen fest mit dem Deckel und/oder dem Rahmen verbunden sind und am Deckel und/oder am Rahmen mittels einer Fassung oder einem Sockel oder einem Halter gehalten werden.

Insbesondere ist der Deckel als öffenbarer Deckel ausgebildet. Der Deckel kann abnehmbar und/oder aufschwenkbar an dem Rahmen gelagert sein.

Sowohl bei der Anordnung der UV-Lichtquellen am Deckel als auch am Rahmen ist der Vorteil, dass die UV-Lichtquellen zur Reparatur oder zur Anpassung der Beleuchtungsstärke durch einfaches Öffnen des Deckels erreicht werden können. Ein Austauschen, Säubern oder Ersetzen der UV-Lichtquellen ist ohne großen Umbau der Vorrichtung schnell und einfach möglich.

Der Vorteil der Anordnung der UV-Lichtquellen am Deckel besteht darin, dass im geöffneten Zustand der Deckel beispielsweise auch als Ablage dienen kann. Zur Reparatur kann der Deckel beispielsweise vollständig abgenommen oder ausgewechselt werden und die UV-Lichtquellen können an einem getrennten Arbeitsplatz auf Ihre Funktion überprüft werden. Auch kann ein Steuergerät der UV-Lichtquelle am Deckel angeordnet sein oder mit dem Deckel verbunden sein und zu Reparaturzwecken an einem getrennten Arbeitsplatz auf Funktion überprüft werden. Ein Vorteil der Anordnung der UV-Lichtquellen direkt am Rahmen besteht darin, dass die mechanisch stabile, insbesondere verwindungsfreie, Befestigung am Rahmen durch die in der Regel größere Stabilität des Rahmens ohne großen Aufwand gewährleistet werden kann.

Es kann vorgesehen sein, dass mehrere Deckel und/oder Wartungsöffnungen im Rahmen ausgebildet sind, wobei in jeder der Wartungsöffnungen jeweils ein Deckel angeordnet ist. Vorzugsweise verschließt jeweils ein Deckel eine Wartungsöffnung.

Es kann vorgesehen sein, dass die einen oder mehreren Wartungsöffnungen am unteren Rahmenabschnitt, am oberen Rahmenabschnitt, am linken Rahmenabschnitt und/oder am rechten Rahmenabschnitt ausgebildet sind. Es kann vorgesehen sein, dass in allen Rahmenabschnitten eine oder mehrere Wartungsöffnungen ausgebildet sind, um die mehreren UV-Lichtquellen zur Reparatur oder zum Auswechseln zu erreichen.

Es kann vorgesehen sein, dass im Bereich eines Deckels, bzw. dass an jedem Deckel eine oder mehrere UV-Lichtquellen angeordnet sind, und/oder dass an jedem Rahmenabschnitt eine oder mehrere UV-Lichtquellen angeordnet sind. Insbesondere kann eine UV-Lichtquelle jeweils einem Deckel und/oder einer Wartungsöffnung zugeordnet sein.

Im Folgenden gilt, wenn ein Deckel und/oder eine Wartungsöffnung durch mögliche Ausgestaltungen spezifiziert wird, ist dies nicht als einschränkend zu verstehen. Es kann dabei vorgesehen sein, dass nur ein Deckel und/oder Wartungsöffnung, oder mehrere Deckel und/oder Wartungsöffnungen oder alle Deckel und/oder Wartungsöffnungen der Vorrichtung derart ausgestaltet sein können.

In einer Ausgestaltung kann vorgesehen sein, dass die Wartungsöffnungen rechteckig mit ihrer Längsseite in Transportrichtung und/oder mit ihrer Querseite in Transportrichtung ausgebildet sind.

Es kann zudem vorgesehen sein, dass der Deckel herausnehmbar oder klappbar ausgebildet ist, vorzugsweise klappbar durch ein Scharnier am Rahmen befestigt ist. Der Vorteil eines klappbaren Deckels ist, dass bei der Reparatur oder dem Wechseln der UV-Lichtquelle der Deckel einfach geöffnet und geschlossen werden kann und damit der Zugang zur UV-Lichtquelle ohne Umbauten der Vorrichtung gewährleistet wird.

Insbesondere kann vorgesehen sein, dass der UV-Tunnel einen Einlassbereich und einen Auslassbereich aufweist, wobei vorzugsweise im Einlassbereich und/oder im Auslassbereich Lichtschleusen ausgebildet sind, insbesondere um einen Austritt von UV-Licht aus dem Tunnel zu verhindern und Bedienpersonal zu schützen.

Ein Vorteil einer solchen Lichtschleuse ist, dass die Umgebung außerhalb des UV-Tunnels frei von UV-Strahlung bleibt und sich Personen gefahrlos während des Betriebs in der Nähe des UV-Tunnels aufhalten können.

Es kann vorgesehen sein, dass die Lichtschleusen einen Vorhang oder eine Wand oder ein Labyrinth aufweisen.

Es kann vorgesehen sein, dass im UV-Tunnel eine schaltbare Sperrvorrichtung ausgebildet ist, welche das Herausnehmen oder das Aufklappen des Deckels bei Betrieb der UV-Lichtquellen sperrt oder freigibt, wenn die UV-Lichtquellen ausgeschaltet sind.

Um die Sicherheit der Vorrichtung zu erhöhen kann vorgesehen sein, dass im UV-Tunnel ein Sensor angeordnet ist, welcher die UV-Bestrahlung detektiert und/oder dass im UV-Tunnel ein Kontaktschalter angeordnet ist, welcher die Stellung des Deckels detektiert und/oder die Offen-Stellung und/oder Geschlossen-Stellung des Deckels detektiert.

Es kann vorgesehen sein, dass der Sensor zur Detektion der UV-Bestrahlung die Sperrvorrichtung sperrt oder freigibt, indem bei vorhandener UV-Strahlung die Sperrvorrichtung zum Sperren angesteuert wird. Alternativ oder ergänzend kann der Sensor die Sperrvorrichtung bei ausgeschalteter UV-Bestrahlung freigeben.

Es kann vorgesehen sein, dass der Sensor oder der Kontaktschalter zur Detektion der Stellung des Deckels und/oder die Offen-Stellung und/oder Geschlossen-Stellung des Deckels die UV-Lichtquelle in Offen-Stellung ausschaltet und/oder in Geschlossen Stellung anschaltet, vorzugsweise dass der Sensor bei der Detektion der Offen-Stellung die UV-Lichtquellen im UV-Tunnel abschaltet.

Um die Betriebsdauer der Vorrichtung zu erhöhen kann vorgesehen sein, dass der UV-Tunnel eine Kühlung aufweist, vorzugsweise eine Luftkühlung, um die UV-Leuchtmittel zu kühlen.

Es kann vorgesehen sein, dass die Kühlung eine Pumpe aufweist, um die Luft durch das Kühlsystem zu pumpen. Zur Kühlung kann Luft aus der Umgebung verwendet werden. Es kann auch Außenluft zur Kühlung verwendet werden, oder eine Mischung aus Umgebungsluft und Außenluft zur Kühlung verwendet werden.

Insbesondere kann die Luft zur Kühlung vorkonditioniert werden, bspw. beheizt werden, um eine Mindesttemperatur zu gewährleisten, oder gekühlt werden, um eine Höchsttemperatur nicht zu überschreiten. Alternativ oder ergänzend kann die Luft zur Kühlung entfeuchtet werden, um einen Feuchtigkeitseintrag in den UV-Tunnel zu verhindern. Auch eine Filterung, um Staubpartikel aus der Luft zur Kühlung zu entfernen, kann vorgesehen sein.

Weiter kann die Kühlung eine Frischluftansaugung und/oder einen Wärmetauscher aufweisen. Es kann vorgesehen sein, dass die Abluft der Kühlung oder ein Teil der Abluft zum Temperieren der Kühlluft verwendet wird, indem über den Wärmetauscher ein Teil der Wärmemenge der Abluft auf die zur Kühlung zugeführte Kühlluft oder die zur Kühlung angesaugte Zuluft oder zur Kühlung angesaugte Außenluft übertragen wird.

Es kann vorgesehen sein, dass der Rahmen und/oder der Deckel untereinander verbundene Hohlprofile aufweist oder als Hohlprofil ausgebildet ist. Vorteilhafterweise sind die Hohlprofile als Luftkanäle zum Transport der Kühlluft innerhalb des Hohlprofils oder der Hohlprofile ausgebildet.

Vorteil einer Ausbildung des Rahmens und/oder des Deckels als Hohlprofil ist der platzsparende Aufbau und die gleichzeitige Transportmöglichkeit der Kühlluft. Es sind keine zusätzlichen Leitungen für die Kühlluft notwendig. Die Hohlprofile bilden zugleich eine tragende Rahmenstruktur und leiten die Kühlluft.

Es kann vorgesehen sein, dass in dem Deckel Hohlprofile zum Transport von Kühlluft vorgesehen sind, oder dass der Deckel als Hohlprofil zum Transport von Kühlluft ausgebildet ist. Insbesondere können Kühllufttransportöffnungen im Rahmen-Hohlprofil und im Deckel-Hohlprofil zum Transport der Kühlluft vom Rahmen in den Deckel ausgebildet sind. Um die Kühlluft von dem Rahmen zum Deckel zu leiten, kann vorgesehen sein, dass im Übergangsbereich eine Kupplung vorgesehen ist, die ein rahmenseitiges Hohlprofil beim Schließen des Deckels automatisch mit einem deckelseitigen Hohlprofil koppelt. Vorzugsweise überlappen die Kühllufttransportöffnungen im Rahmen-Hohlprofil und im Deckel-Hohlprofil bei geschlossenem Deckel, um einen durchgehenden Kühllufttransport zu ermöglichen.

Vorzugsweise ist das Gestell als Hohlprofil ausgebildet zum Transport der Kühlluft. Es kann vorgesehen sein, dass Kühllufttransportöffnungen im Gestell-Hohlprofil und im Deckel-Hohlprofil zum Transport der Kühlluft vom Gestell in den Deckel ausgebildet sind. Vorzugsweise überlappen die Kühllufttransportöffnungen im Gestell-Hohlprofil und im Deckel-Hohlprofil bei geschlossenem Deckel.

Es kann vorgesehen sein, dass im Deckel oder im Hohlprofil im Bereich des Deckels eine oder mehrere Kühlluftausgangsöffnungen ausgebildet sind, vorzugsweise dass die Kühlluftausgangsöffnungen in Richtung des UV-Leuchtmittels verlaufend ausgebildet sind, um das UV-Leuchtmittel durch aus dem Deckel oder Hohlprofil austretende Kühlluft zu kühlen.

Es kann vorgesehen sein, dass die UV-Leuchtmittel über elektrische Leitungen mit einem Steuergerät und/oder einem Vorschaltgerät und/oder einem Zündgerät und/oder der Sperrvorrichtung verbunden sind, vorzugsweise dass das Steuergerät und/oder das Vorschaltgerät und/oder das Zündgerät und/oder die Sperrvorrichtung außerhalb des UV-Tunnels angeordnet ist bzw. sind.

Es kann vorgesehen sein, dass elektrische Leitungen zum Anschluss der UV-Leuchtmittel innerhalb eines Hohlprofils des Rahmens und/oder des Deckels angeordnet bzw. geführt sind. Beispielsweise kann ein Hohlprofil des Deckels oder des Rahmens gleichzeitig zur Aufnahme einer elektrischen Leitung und zum Transport von Kühlluft ausgebildet sein. Alternativ oder ergänzend können elektrische Leitungen an der Innenseite des Rahmens und/oder des Deckels angeordnet sein.

Es kann vorgesehen sein, dass zwischen Rahmen und Deckel eine elektrische Leitung ausgebildet ist. Beispielsweise kann eine Kabelschleife vorgesehen sein, um ein Öffnen des Deckels zu ermöglichen. Es kann auch vorgesehen sein, dass zwischen Rahmen und Deckel elektrische Kontaktstifte oder Kontaktflächen ausgebildet sind und durch Schließen des Deckels ein elektrischer Kontakt zwischen einer elektrischen Leitung im Deckel und einer elektrischen Leitung im Rahmen ausgebildet wird, vorzugsweise dass der Kontakt beim Öffnen des Deckels automatisch unterbrochen wird.

Es kann vorgesehen sein, dass die Innenseite des UV-Tunnels zum Teil oder vollständig mit einer UV-reflektierenden Beschichtung versehen ist, insbesondere mit Hochglanzaluminium ausgekleidet ist. Dadurch kann die von den UV-Lichtquellen generierte UV-Strahlung effizient genutzt werden bzw. in Richtung auf die zu desinfizierenden Pakete oder Stückgut geleitet werden.

Es kann vorgesehen sein, dass auf der Rückseite der UV-Leuchtmittel Reflektoren angeordnet sind oder die UV-Leuchtmittel in Scheinwerfern mit Reflektoren und/oder Linsen angeordnet sind, vorzugsweise um die UV-Strahlung der UV-Leuchtmittel auf die Pakete oder das Stückgut zu richten.

Es kann vorgesehen sein, dass mindestens zwei UV-Leuchtmittel an einem Deckel angeordnet sind, und/oder dass mindestens zwei, vier oder sechs Wartungsöffnungen pro Rahmenabschnitt ausgebildet sind.

Es kann vorgesehen sein, dass der Rahmen des UV-Tunnels an seiner Innenseite mit dem unteren Rahmenabschnitt, dem oberen Rahmenabschnitt, dem linken Rahmenabschnitt und dem rechten Rahmenabschnitt ein Rechteck ausbildet, durch welches das Transportband die Paketsendungen hindurchführt, vorzugsweise dass das Rechteck mindestens 400 mm x 400 mm groß ist, vorzugsweise mindestens 600 mm x 600 mm groß ist, mindestens 800 mm x 800 mm groß ist.

Es kann vorgesehen sein, dass der Deckel eine Länge von mindestens 1,5 m aufweist. Der Vorteil der Länge besteht darin, dass UV-Leuchtmittel mit einer Standardlänge von 1,47 m durch den Deckel hindurch ausgetauscht werden können oder an der Innenseite des Deckels angeordnet werden können und der Deckel ohne große bauliche Maßnahmen geöffnet oder entfernt werden kann.

Es kann vorgesehen sein, dass im UV-Tunnel mehrere Wartungsöffnungen und Deckel in Transportrichtung hintereinander ausgebildet sind. Dadurch kann die Beleuchtungszeit der Pakete erhöht werden oder die Transportgeschwindigkeit der Paketsendung erhöht werden. Weiter wird durch einen längeren UV-Tunnel ein Paket auch an der Vorderseite und Rückseite bestrahlt, wobei die Vorderseite und Rückseite eines Pakets oder Stückguts den Seiten in Transportrichtung und gegen die Transportrichtung entspricht. Vorzugsweise werden durch Scheinwerfer die UV-Strahlen oder ein Teil der UV-Strahlen auf die Vorderseite und die Rückseite eines Pakets gerichtet.

Es kann vorgesehen sein, dass der UV-Tunnel als modularer UV-Tunnel ausgebildet ist. Insbesondere kann der UV-Tunnel aus einem oder zwei oder mehreren gleich aufgebauten Modulen ausgebildet sein. Insbesondere weist ein Modul jeweils einen Modul-Rahmen mit einem unteren Modul-Rahmenabschnitt, einem oberen Modul-Rahmenabschnitt, einem linken Modul-Rahmenabschnitt und einem rechten Modul-Rahmenabschnitt auf. Insbesondere schließen die mehreren Module des UV-Tunnels in Transportrichtung unmittelbar aneinander an, bzw. sind so miteinander verbunden, dass zwischen den Modulen kein UV-Licht in die Umgebung austritt.

Der Vorteil eines modular ausgebildeten UV-Tunnels liegt in der Skalierbarkeit der Vorrichtung, d.h. die Länge des UV-Tunnels kann angepasst werden. Die Module, welche vorzugsweise denselben Querschnitt aufweisen, können fluchtend hintereinander in Längsrichtung angeordnet werden und so den UV-Tunnel verlängern. Es kann vorgesehen sein, dass die Module in Transportrichtung in Standardlängen ausgebildet sind, z.B. abhängig von den UV-Leuchtmitteln. Durch die Module kann der UV-Tunnel an die baulichen Gegebenheiten angepasst werden oder and die Geschwindigkeit des Transportbandes angepasst werden. Beispielsweise kann bei schnellerer Transportgeschwindigkeit ein längerer UV-Tunnel vorgesehen werden, um die Beleuchtungssituation anzupassen.

Vorzugsweise weist jedes Modul in Transportrichtung gesehen nur ein UV-Leuchtmittel auf, d.h. in Transportrichtung sind in einem Modul nicht mehrere UV-Leuchtmittel hintereinander ausgebildet.

Es kann vorgesehen sein, dass mehrere Module hintereinander angeordnet sind, um den UV-Tunnel zu verlängern, vorzugsweise derart hintereinander angeordnet sind, dass mehrere UV-Leuchtmittel in Transportrichtung hintereinander angeordnet sind.

Es kann vorgesehen sein, dass die Vorrichtung zum Desinfizieren und/oder Sterilisieren von Paketen oder Stückgut mittels UV-Licht aus einem Bausatz besteht umfassend mehrere Module und ein Transportband, wobei der UV-Tunnel durch die mehreren Module gebildet wird.

Es kann vorgesehen sein, dass die mehreren Module einen gemeinsamen Rahmen aufweisen oder jedes Modul einen separaten Rahmen aufweist.

Vorzugsweise sind in den separaten Rahmen Kühllufttransportöffnungen im Hohlprofil ausgebildet um die Kühlluft zwischen den Modulen zu übertragen. Der Vorteil des modularen Aufbaus mit Kühllufttransportöffnungen zwischen den Modulen besteht darin, dass nur eine Luftquelle ausgebildet sein muss, um die UV-Leuchtmittel in allen Modulen zu kühlen.

Es kann vorgesehen sein, dass die UV-Lichtquelle eine oder mehrere UV-Röhren oder eine oder mehrere UV-Leuchtstoffröhren oder eine oder mehrere UV-Leuchtdioden (LED) aufweist oder als solche ausgebildet ist.

Es kann vorgesehen sein, dass eine UV-Lichtquelle eine Leistung von mindestens 100 W, vorzugweise mindestens 300 W aufweist.

Es kann vorgesehen sein, dass das Transportband aus mehreren geschlossen umlaufenden Riemen, vorzugsweise Zahnriemen ausgebildet ist, vorzugsweise dass die Riemen oder die Zahnriemen quer zur Transportrichtung zueinander beabstandet angeordnet und gemeinsam als Transportband ausgebildet sind.

Mit Abstand zueinander als Transportband ausgebildet bedeutet, dass die Riemen oder Zahnriemenmit mit seitlichem Abstand zueinander angeordnet sind. Der Vorteil des Abstands besteht darin, dass die freien Bereiche von unten mit UV-Licht bestrahlet werden können. Der Vorteil eines Transportbands als Zahnriemen ist, dass der Antrieb durch Kraft- und Formschluss auf den Zahnriemen übertragen wird und somit auch schwere Pakete sicher transportiert werden können. Vorzugsweise kann zur Unterstützung der Riemen zum Transport der Pakete oder des Stückguts und zur Führung des Riemens auf übereinanderliegenden Ebenen ein Obertrum und/oder ein Untertrum ausgebildet sein, vorzugsweise als Tragerolle oder Trageschiene.

Es kann vorgesehen sein, dass das Transportband in dem UV-Tunnel bzw. in einem Modul des UV-Tunnels aus zwei Abschnitten ausgebildet ist, einem vorderen Transportband und einem hinteren Transportband, vorzugsweise sind die zwei Transportbänder als getrennte Transportbänder ausgebildet.

Es kann vorgesehen sein, dass das vordere Transportband und das hintere Transportband aus Riemen, vorzugsweise Zahnriemen ausgebildet sind, vorzugsweise dass die Riemen oder Zahnriemen mit Abstand zueinander als Transportband ausgebildet sind.

Es kann vorgesehen sein, dass die Riemen, vorzugsweise Zahnriemen, des vorderen Transportbands mit Abstand zueinander ausgebildet sind, und
dass die Riemen, vorzugsweise Zahnriemen, des hinteren Transportbands mit Abstand zueinander ausgebildet sind, und
dass die Riemen, vorzugsweise Zahnriemen, des vorderen Transportbands versetzt zu den Riemen, vorzugsweise Zahnriemen, des hinteren Transportband ausgebildet sind.

Unter versetzt zueinander ist zu verstehen, dass ein Riemen des vorderen Transportbands in einer ersten, parallel zu der Transportrichtung verlaufenden Ebene angeordnet ist und in dieser ersten Ebene im hinteren Transportband kein Riemen angeordnet ist.

Insbesondere sind unterhalb des Transportbandes eine oder mehrere UV-Lichtquellen angeordnet, um die Unterseite eines Pakets oder eines Stückguts mit UV-Licht zu bestrahlen und zu desinfizieren. Der Vorteil einer derartigen Ausgestaltung besteht darin, dass durch die versetzte und beabstandete Ausbildung der Riemen, vorzugsweise der Zahnriemen, eine Paketsendung oder ein Stückgut auch von unten vollständig mit UV-Licht bestrahlt werden kann. Insbesondere wird eine partielle Abschattung der Unterseite durch das Transportband oder die Riemen verhindert.

Es kann vorgesehen sein, dass das Transportband einen Motor aufweist, der das Transportband antreibt, vorzugsweise dass das vordere Transportband und das hintere Transportband einen gemeinsamen Motor aufweisen, der beide Transportbänder antreibt.

Es kann vorgesehen sein, dass die beiden Transportbänder über eine gemeinsame Antriebsache angetrieben werden, vorzugsweise dass die gemeinsame Antriebsachse am Kreuzungspunkt der beiden Transportbänder angeordnet ist.

Es kann vorgesehen sein, dass die Oberfläche des Riemens, vorzugsweise des Zahnriemens, UV-beständig ausgebildet, vorzugsweise UV-beständig beschichtet ist.

Es kann vorgesehen sein, dass der Motor außerhalb des UV-Tunnels angeordnet ist, vorzugsweise dass der Motor und die Antriebsachse über ein Getriebe miteinander verbunden sind.

Es kann vorgesehen sein, dass im UV-Tunnel ein Anschlag oder eine Seitenführung parallel zur Transportrichtung ausgebildet ist, um ein Anstoßen eines Pakets und/oder eines Stückguts an das seitliche UV-Leuchtmittel zu verhindern, und/oder um eine Paketsendung und/oder ein Stückgut mittig auf dem Transportband auszurichten bzw. zu halten, oder um zu allen Seiten des UV-Tunnels einen vorgegebenen Abstand bzw. einen minimalen Mindest-Abstand zu gewährleisten.

Es kann vorgesehen sein, dass zwei oder mehrere Anschläge oder Seitenführungen in der Höhe versetzt zueinander ausgebildet sind, wobei sich die mehreren Anschläge in Transportrichtung nur teilweise überlappen, um die Seitenfläche einer Paketsendung vollständig von der Seite zu Beleuchten und Abschattungen zu vermeiden.

Eine Anwendung der erfindungsgemäßen Vorrichtung zum Desinfizieren und/oder Sterilisieren von Paketen oder Stückgut kann beispielsweise in Verteilzentren von Versanddienstleistern oder in einem automatisierten Lager in der Fertigung erfolgen.

Weitere Ausführungen der Erfindung sind in den Figuren dargestellt und nachfolgend beschrieben. Dabei zeigen:
- Fig. 1:: Schematische Darstellung der erfindungsgemäßen Vorrichtung zum Desinfizieren und/oder Sterilisieren von Paketen oder Stückgut mittels UV-Licht;
- Fig. 2:: Draufsicht der Vorrichtung aus Fig. 1;
- Fig. 3:: Sicht von unten der Vorrichtung aus Fig. 1;
- Fig. 4:: Schematische Darstellung eines Transportbandes der Vorrichtung aus Fig. 1 mit mehreren beabstandeten Riemen.

In den Figuren ist beispielhaft eine mögliche Ausgestaltung der Erfindung gezeigt. Diese Ausgestaltung dient der Erläuterung einer möglichen Umsetzung der Erfindung und soll nicht eingrenzend verstanden werden.

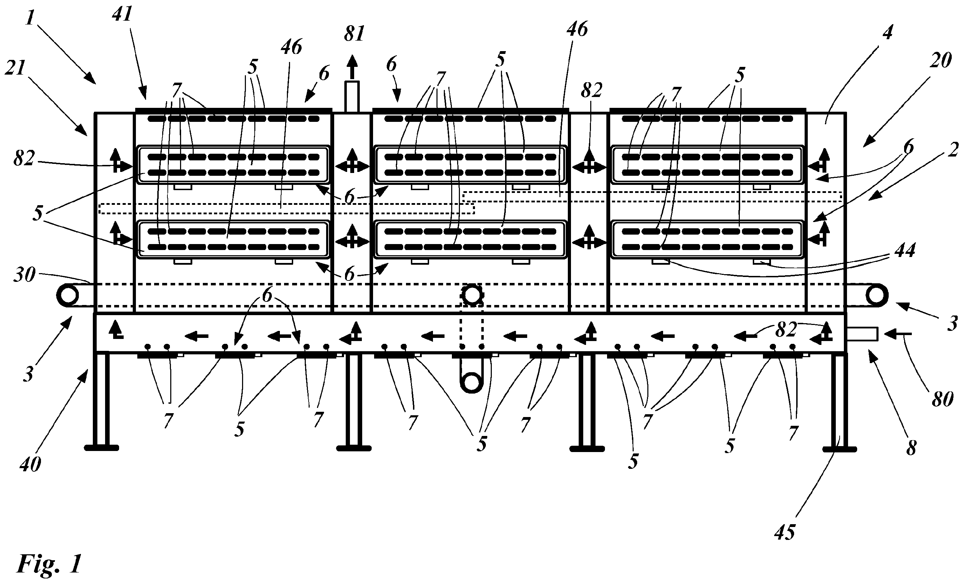

Fig. 1 zeigt die Vorrichtung 1 zum Desinfizieren und/oder Sterilisieren von Paketen oder Stückgut mittels UV-Licht. Die Vorrichtung umfasst einen UV-Tunnel 2 und ein durch den UV-Tunnel 2 hindurch geführtes Transportband 3. Auf dem Transportband 3 können Pakete und/oder Stückgut durch den UV-Tunnel 2 hindurch transportiert und dabei mit UV-Licht bestrahlt werden. Der UV-Tunnel 2 ist im Ausführungsbeispiel der Fig. 1 auf Füßen 45 angeordnet.

Der UV-Tunnel 2 im Ausführungsbeispiel der Fig. 1 umfasst einen Rahmen 4, welcher mit seinem unteren Rahmenabschnitt 40, seinem oberen Rahmenabschnitt 41, seinem linken Rahmenabschnitt 42 und seinem rechten Rahmenabschnitt den UV-Tunnel 2 ausbildet (siehe Fig. 1, 2 und 3). Der Rahmen 4 mit seinem Rahmenabschnitten umschließt das Transportband 3 von vier Seiten, so dass der UV-Tunnel 2 einen Tunnel mit einem offenen Einlassbereich 20 und einem offenen Auslassbereich 21 ausbildet.

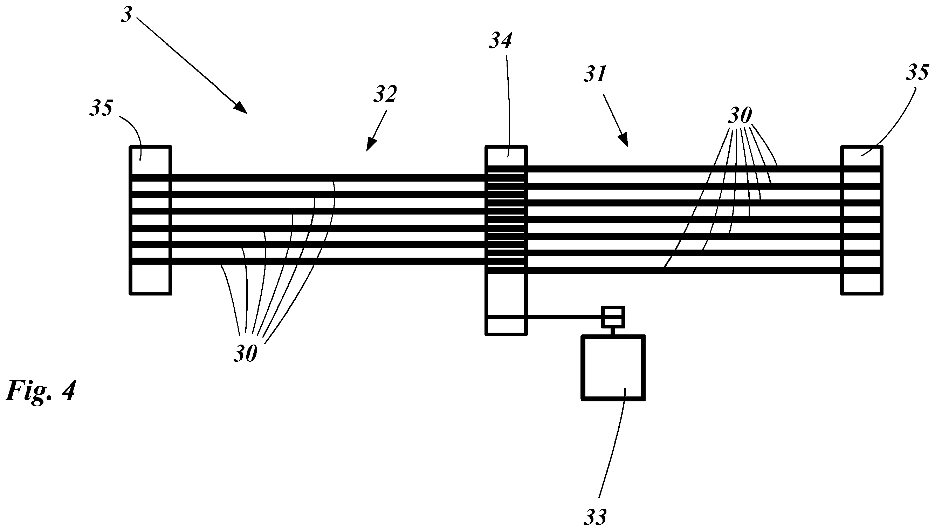

Im Ausführungsbeispiel der Fig. 1 ist das Transportband 3 als zwei-stückiges Transportband 3 ausgebildet und steht im Einlassbereich 20 und im Auslassbereich 21 über den UV-Tunnel 2 hinaus. Das Transportband 3 wird in Fig. 4 im Detail beschrieben.

In jedem der Rahmenabschnitte 40, 41, 42, 43 sind Wartungsöffnungen 6 ausgebildet, welche durch jeweils einen Deckel 5 verschlossen werden können. D.h. wenn ein Deckel 5 in der Wartungsöffnung 6 angeordnet ist, und diese vollständig abdeckt, ist dies die Geschlossen-Stellung des Deckels 5. Eine abgenommener Deckel 5, welcher nicht in der Wartungsöffnung 6 angeordnet ist und diese nicht vollständig abdeckt bildet die Offen-Stellung des Deckels 5.

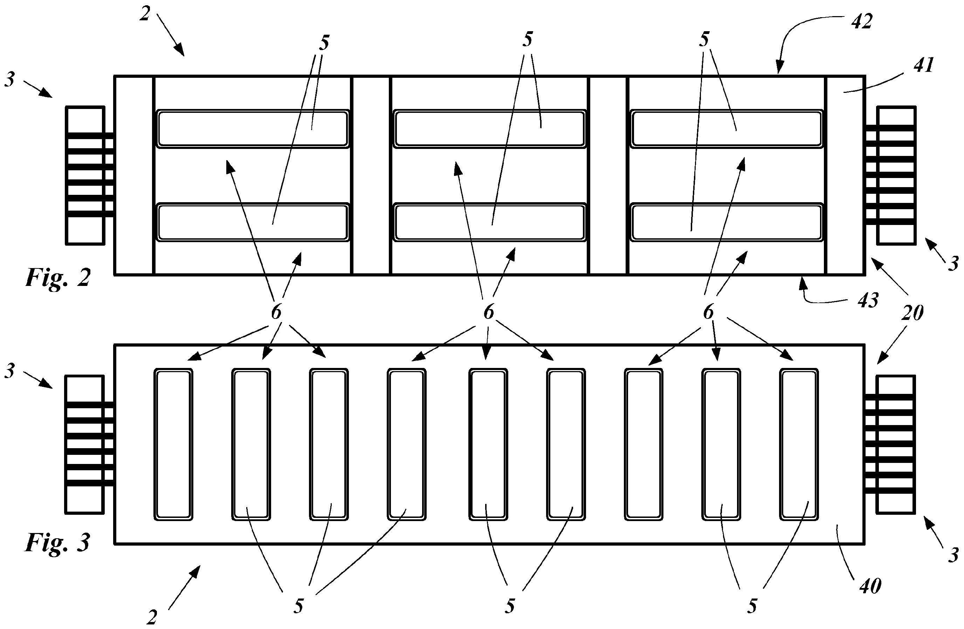

Im Ausführungsbeispiel der Fig. 1 sind jeweils sechs Wartungsöffnungen 6 im oberen Rahmenabschnitt 41 (siehe Fig. 2), im linken Rahmenabschnitt 42 und im rechten Rahmenabschnitt 43 (siehe Fig. 1) als rechteckige Öffnungen ausgebildet, welche sich mit ihrer Längserstreckung in die Transportrichtung vom Einlassbereich 20 in Richtung des Auslassbereichs 21 erstrecken. Jede dieser Wartungsöffnungen 6 ist durch einen Deckel 5 abgedeckt. Im unteren Rahmenabschnitt 42 sind neun Wartungsöffnungen 6 als rechteckige Öffnungen, mit ihrer Längserstreckung rechtwinklig zur Transportrichtung, ausgebildet. Die Deckel 5 sind im Ausführungsbeispiel der Fig. 1 über jeweils zwei Scharniere 44 aufklappbar mit dem Rahmen 4 verbunden. Im geschlossenen Zustand der Deckel 5 werden die Wartungsöffnungen 6 vollständig abgedeckt, um ein Austreten von UV-Strahlung zu verhindern.

Im Ausführungsbeispiel der Fig. 1 sind an jeder Wartungsöffnung 6 jeweils zwei UV-Lichtquellen 7 angeordnet, welche in Fig. 1 als dicke gestrichelte Linien dargestellt sind, bzw. als Punkte im unteren Rahmenabschnitt 40. Die Anordnung und Erstreckung der UV-Lichtquellen 7 ist analog zur Ausbildung der Wartungsöffnungen 6 in Richtung der Transportrichtung oder im unteren Rahmenabschnitt 40 im rechten Winkel dazu. Die UV-Lichtquellen 7 können dabei entweder am Rahmen 4 oder an den Deckeln 5 befestigt sein. Bei einer Befestigung der UV-Lichtquellen 7 an den Deckeln 5 würden die UV-Lichtquellen 7 beim Öffnen der Deckel 5 aus der Wartungsöffnung 6 heraus bewegt werden.

Um ein Anstoßen der Pakete oder des Stückguts an den seitlichen UV-Lichtquellen 7 zu verhindern, sind im UV-Tunnel 2 jeweils auf der linken und rechten Seite, d.h. am linken Rahmenabschnitt 42 und am rechten Rahmenabschnitt 43 Seitenführungen 46 angeordnet, welche über die UV-Lichtquellen 7 in den UV-Tunnel 2 hinein überstehen. Die Seitenführungen 46 bestehen dabei aus zwei Anschlag- und Führungsbalken, welche in der Höhe versetzt zueinander angeordnet sind, um ein Bestrahlen der Pakete oder des Stückguts über die gesamte Seitenfläche zu ermöglichen.

Die Fig. 3 und 4 zeigen den UV-Tunnel 2 und das Transportband 3 von einer Draufsicht und einer Sicht von unten, wobei von unten die Füße 45 aus Fig. 1 nicht dargestellt sind. In Fig. 3 sind die rechteckigen Wartungsöffnungen 6 und die Deckel 5 in ihrer Längserstreckung in Transportrichtung ausgebildet, wobei in Fig. 4 auf der Unterseite des UV-Tunnels 2 die rechteckigen Wartungsöffnungen 6 und die Deckel 5 in ihrer Längserstreckung rechtwinklig zur Transportrichtung ausgebildet sind.

Um die UV-Lichtquellen 7 zu kühlen ist im Ausführungsbeispiel der Fig. 1 eine Kühlung 8 vorgesehen. Durch einen Kühlungseingang 80 wird kalte Luft in den Rahmen 4 eingeführt. Der Rahmen 4 ist in diesem Ausführungsbeispiel als ein Hohlprofil ausgebildet, in dem sich die kalte Luft im Rahmen in die Ausbreitungsrichtungen 82 (schwarze Pfeile im Rahmen 4 in Fig. 1) vollständig im Rahmen 4 über den gesamten UV-Tunnel ausbreiten kann. Die warme Luft kann über einen Kühlungsausgang aus dem UV-Tunnel 2 entweichen. In dem Rahmen 4 und in den Deckeln 5 sind Öffnungen (nicht dargestellt in Fig. 1) vorgesehen, welche eine Luftverbindung zwischen Rahmen 4 und Deckel 5 ausbilden, wodurch sich die kalte Luft, wie im Rahmen 4, im Hohlprofil der Deckel 5 ausbreiten kann. Im Deckel 5 sind zusätzlich Kühlluftausgangsöffnungen ausgebildet, welche die kühle Luft von hinten, und/oder von oben, und/oder von unten auf die UV-Lichtquellen 7 leiten.

In Fig. 4 ist das Transportband 3 der Vorrichtung 1 aus Fig. 1 dargestellt. Das Transportband 3 setzt sich in diesem Ausführungsbeispiel aus einem vorderen Transportband 31 und einem hinteren Transportband 32 zusammen. Jedes Transportband 31, 32 setzt sich aus mehreren Riemen 30 zusammen, welche mit Abstand zueinander zwischen einer Antriebsrolle 34 und einer Umlenkrolle 35 auf Spannung gehalten werden. Im Ausführungsbeispiel der Fig. 4 teilen sich die beiden Transportbänder 31 und 32 eine Antriebsrolle 34, wobei die Antriebsrolle 34 über einen Motor 33 angetrieben wird. Weiter sind die Riemen 30 des ersten Transportbands 31 versetzt zu den Riemen 30 des zweiten Transportbands 32 gelagert. Die Abstände zwischen den Riemen 30 und die versetzte Lagerung der Riemen 30 der beiden Transportbänder 31 und 32 erlaubt ein Bestrahlen eines Pakets oder eines Stückguts von unten über den vollständigen Bodenbereich des Pakets oder des Stückguts.

### Bezugszeichenliste

- 1: Vorrichtung zum Desinfizieren und/oder Sterilisieren
- 2: UV-Tunnel
- 3: Transportband
- 4: Rahmen
- 5: Deckel
- 6: Wartungsöffnung
- 7: UV-Lichtquelle
- 8: Kühlung
- 20: Einlassbereich
- 21: Auslassbereich
- 30: Riemen
- 31: vorderes Transportband
- 32: hinteres Transportband
- 33: Motor
- 34: Antriebsrolle
- 35: Umlenkrolle
- 40: unterer Rahmenabschnitt
- 41: oberer Rahmenabschnitt
- 42: linker Rahmenabschnitt
- 43: rechter Rahmenabschnitt
- 44: Scharnier
- 45: Fuß
- 46: Seitenführung
- 80: Kühlungseingang
- 81: Kühlungsausgang

## Patentansprüche

1. Vorrichtung (1) zum Desinfizieren und/oder Sterilisieren von Paketen oder Stückgut mittels UV-Licht, umfassend einen UV-Tunnel (2) und ein durch den UV-Tunnel (2) in einer Transportrichtung hindurch geführtes Transportband (3) zum Transport von Paketen oder Stückgut,
wobei innerhalb des UV-Tunnels (2) mehrere UV-Lichtquellen (7) angeordnet sind, und
wobei der UV-Tunnel (2) einen Rahmen (4) mit einem in Transportrichtung gesehenen unteren Rahmenabschnitt (40), einem oberen Rahmenabschnitt (41), einem linken Rahmenabschnitt (42) und einem rechten Rahmenabschnitt (43) aufweist, und die Rahmenabschnitte das Transportband (3) an vier Seiten umschließen,
**dadurch gekennzeichnet,**
**dass** in oder an dem Rahmen (4) eine durch einen öffenbaren oder abnehmbaren Deckel (5) verschlossene Wartungsöffnung (6) ausgebildet ist,
wobei der Deckel (5) derart ausgebildet und angeordnet ist, dass er in geschlossenem Zustand die Wartungsöffnung (6) vollständig verschließt und in geöffnetem oder abgenommenem Zustand einen Zugriff auf eine oder mehrere oder alle UV-Lichtquellen (7) und/oder einen Austausch einer oder mehrerer oder aller UV-Lichtquellen (7) ermöglicht, und
**dass** die mehreren UV-Lichtquellen (7) die Pakete oder das Stückgut von vier Seiten mit UV-Licht bestrahlen.

2. Vorrichtung (1) zum Desinfizieren und/oder Sterilisieren von Paketen oder Stückgut mittels UV-Licht nach Anspruch 1,
**dadurch gekennzeichnet,**
**dass** die UV-Lichtquellen (7) im UV-Tunnel (2) an der Innenseite des Deckels (5) angeordnet sind, vorzugsweise mit der Innenseite des Deckels (5) mechanisch verbunden sind, und/oder
**dass** die UV-Lichtquellen (7) im UV-Tunnel (2) an der Innenseite des Rahmens (4) angeordnet sind, vorzugsweise am unteren Rahmenabschnitt (40), am oberen Rahmenabschnitt (41), am linken Rahmenabschnitt (42) und/oder am rechten Rahmenabschnitt (43) angeordnet sind.

3. Vorrichtung (1) zum Desinfizieren und/oder Sterilisieren von Paketen oder Stückgut mittels UV-Licht nach Anspruch 1,
**dadurch gekennzeichnet,**
**dass** mehrere Wartungsöffnungen (6) im Rahmen (4) ausgebildet sind, wobei in jeder der Wartungsöffnungen (6) jeweils ein Deckel (5) angeordnet ist.

4. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** der Deckel (5) herausnehmbar oder klappbar ausgebildet ist, vorzugsweise durch ein Scharnier (44) am Rahmen (4) befestigt ist.

5. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** der UV-Tunnel (2) einen Einlassbereich (20) und einen Auslassbereich (20) aufweist, wobei vorzugsweise im Einlassbereich (20) und/oder im Auslassbereich (20) Lichtschleusen ausgebildet sind.

6. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** im UV-Tunnel (2) eine schaltbare Sperrvorrichtung ausgebildet ist, welche das Herausnehmen oder das Aufklappen des Deckels (5) bei Betrieb der UV-Lichtquellen (7) sperrt oder freigibt, wenn die UV-Lichtquellen (7) ausgeschaltet sind.

7. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** im UV-Tunnel (2) ein Sensor angeordnet ist, welcher die UV-Bestrahlung detektiert und/oder dass im UV-Tunnel (2) ein Kontaktschalter angeordnet ist, welcher die Stellung des Deckels (5) detektiert und/oder die Offen-Stellung und/oder Geschlossen-Stellung des Deckels (5) detektiert.

8. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** der UV-Tunnel (2) eine Kühlung aufweist, vorzugsweise eine Luftkühlung, um die UV-Leuchtmittel zu kühlen.

9. Vorrichtung (1) zum Desinfizieren und/oder Sterilisieren von Paketen oder Stückgut mittels UV-Licht nach Anspruch 8,
**dadurch gekennzeichnet,**
**dass** der Rahmen (4) und/oder der Deckel (5) untereinander verbundene Hohlprofile aufweist oder als Hohlprofil ausgebildet ist, vorzugsweise zum Transport der Kühlluft innerhalb des Hohlprofils oder der Hohlprofile.

10. Vorrichtung (1) zum Desinfizieren und/oder Sterilisieren von Paketen oder Stückgut mittels UV-Licht nach Anspruch 8 oder 9,
**dadurch gekennzeichnet,**
**dass** im Deckel (5) oder im Hohlprofil im Bereich des Deckels (5) eine oder mehrere Kühlluftausgangsöffnungen ausgebildet sind, vorzugsweise dass die Kühlluftausgangsöffnungen in Richtung des UV-Leuchtmittels verlaufend ausgebildet sind, um das UV-Leuchtmittel durch aus dem Deckel (5) oder Hohlprofil austretende Kühlluft zu kühlen.

11. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** der UV-Tunnel (2) aus einem oder mehreren gleich aufgebauten Modulen ausgebildet ist, wobei ein Modul jeweils einen Modul-Rahmen mit einem unteren Modul-Rahmenabschnitt, einem oberen Modul-Rahmenabschnitt, einem linken Modul-Rahmenabschnitt und einem rechten Modul-Rahmenabschnitt aufweist, wobei vorzugsweise vorgesehen ist, dass die mehreren Module einen gemeinsamen Rahmen (4) aufweisen oder jedes Modul einen separaten Rahmen (4) aufweist.

12. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** die UV-Lichtquelle (7) eine oder mehrere UV-Röhren oder eine oder mehrere UV-Leuchtstoffröhren oder eine oder mehrere UV-Leuchtdioden (LED) aufweist oder als solche ausgebildet ist.

13. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** das Transportband (3) aus mehreren geschlossen umlaufenden Riemen (30), vorzugsweise Zahnriemen ausgebildet ist, vorzugsweise dass die Riemen (30) oder die Zahnriemen quer zur Transportrichtung zueinander beabstandet angeordnet und als Transportband (3) ausgebildet sind.

14. Vorrichtung (1) zum Desinfizieren und/oder Sterilisieren von Paketen oder Stückgut mittels UV-Licht nach Anspruch 13,
**dadurch gekennzeichnet,**
**dass** das Transportband (3) aus zwei Abschnitten ausgebildet ist, einem vorderen Transportband (31) und einem hinteren Transportband (32), vorzugsweise sind die zwei Transportbänder (31, 32) als getrennte Transportbänder (31, 32) ausgebildet, wobei vorzugsweise vorgesehen ist,
**dass** die Riemen (30), vorzugsweise Zahnriemen, des vorderen Transportbands (31) mit Abstand zueinander ausgebildet sind, und
**dass** die Riemen (30), vorzugsweise Zahnriemen, des hinteren Transportbands (32) mit Abstand zueinander ausgebildet sind, und
**dass** die Riemen (30), vorzugsweise Zahnriemen, des vorderen Transportbands (31) versetzt zu den Riemen (30), vorzugsweise Zahnriemen, des hinteren Transportband (32) ausgebildet sind.

15. Vorrichtung (1) zum Desinfizieren und/oder Sterilisieren von Paketen oder Stückgut mittels UV-Licht nach einem der vorangehenden Ansprüche,
**dadurch gekennzeichnet,**
**dass** das Transportband (3) einen Motor (33) aufweist, der das Transportband (3) antreibt, vorzugsweise dass das vordere Transportband (31) und das hintere Transportband (32) einen gemeinsamen Motor (33) zum Antrieb aufweisen.
